# FASCICULE DE BREVET EUROPEEN

(11) **EP 0 448 684 B1**
(45) Date de publication et mention de la délivrance du brevet: **08.06.1994**
(21) Numéro de dépôt: 90915496.5
(22) Date de dépôt: 09.10.1990
(51) Int. Cl.: A63B 23/08, A63B 24/00

(54) **APPAREIL D'ETUDE ET DE REEDUCATION DE LA CHEVILLE**
VORRICHTUNG ZUM TRAINING DER FUSSKNÖCHEL
APPARATUS FOR STUDYING AND REEDUCATING THE ANKLE

(30) Priorité: 13.10.1989 FR 8913651
(43) Date de publication de la demande: 02.10.1991
(73) Titulaire: HEURTE, Alain, F-29239 Gouesnou (FR)
(72) Inventeur: HEURTE, Alain, F-29239 Gouesnou (FR)
(74) Mandataire: Le Guen, Louis François
(86) Numéro de dépôt international: FR9000719
(87) Numéro de publication internationale: WO9105587

(56) Documents cités:
- CA-A- 1 021 818
- GB-A- 1 372 342
- US-A- 4 463 946
- US-A- 4 705 271

## Description

La présente invention concerne un appareil d'étude et de rééducation de la cheville.

En ce qui concerne les appareils de rééducation de la cheville, l'état de la technique peut être illustré par les documents DE-A-227 571, GB-A-1 372 342, FR-A-2 502 492, FR-A-2 535 209, US-A-4 605 220 et la demande de brevet français n° 88 1-2928 déposée le 28/09/88 par le présent demandeur (FR-A-2 641 183). Tous ces appareils comprennent un plateau monté pivotant sur une rotule ou sur une sorte de demi-boule. Ils permettent notamment de faire récupérer à une cheville pathologique ses propriétés propioceptives, mais ils ne donnent au rééducateur et au patient que des informations subjectives; le rééducateur regarde la cheville réagir et se fie à son expérience personnelle et le patient se fie aux sensations qu'il éprouve.

Le document CA-A-1 021 818 décrit un appareil de rééducation de la cheville comportant un plateau pivotant sur une rotule et des moyens d'enregistrement analogiques des mouvements du plateau.

Le document US-A-4 463 946 décrit un appareil comportant un plateau qui peut basculer autour d'un axe horizontal, le plateau étant destiné à servir de support commun aux deux pieds d'une personne.

Un objet de l'invention consiste à prévoir un appareil qui apporte des informations numériques interprétables notamment par le rééducateur. Les informations numériques obtenues par l'appareil de l'invention permettent, d'une part, de comparer l'état de la cheville pathologique avec celui de la cheville saine du patient et, d'autre part, de suivre l'évolution de la thérapie appliquée.

Cet objet est atteint par la caractéristique de la revendication 1. Les caractéristiques mentionnées dans les revendications secondaires 2 à 4 définissent d'autres applications.

Les caractéristiques de l'invention mentionnées ci-dessus, ainsi que d'autres, apparaîtront clairement à la lecture de la description d'un exemple de réalisation, ladite description étant faite en relation avec les dessins joints, parmi lesquels:

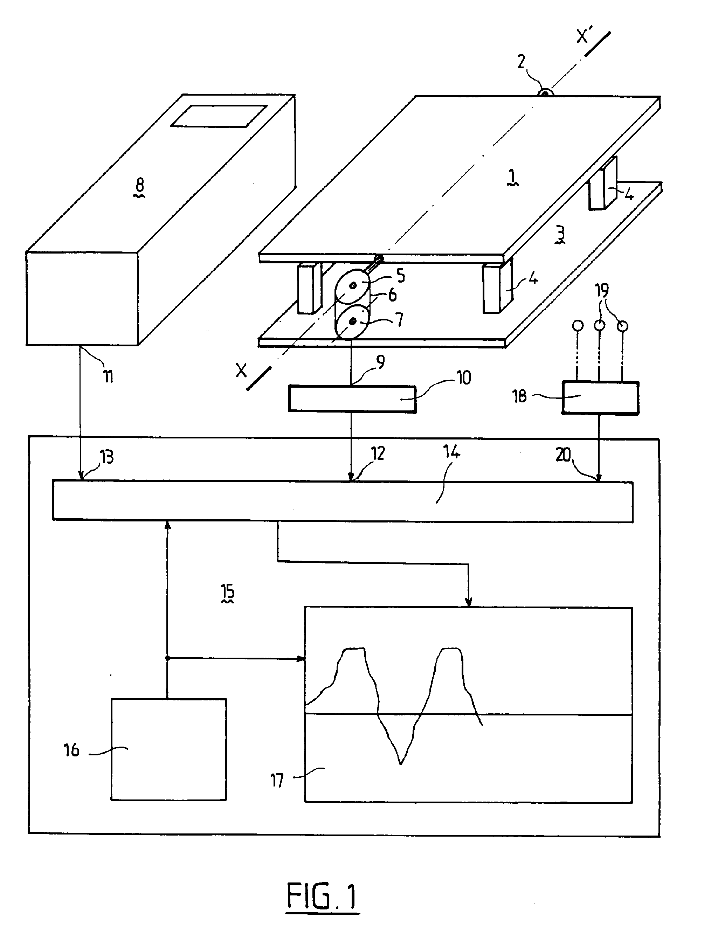

la Fig. 1 est une vue schématique de l'appareil de l'invention.

L'appareil de la Fig. 1 comprend un plateau 1 monté sur un axe horizontal X-X′, lui-même monté sur des potelets porteurs 2, dont un seul est visible, dont les pieds sont fixés sur un socle 3. Des butées verticales 4 partent du socle 3 sous le plateau 1 pour en limiter l'inclinaison autour de l'axe X-X′. Les butées 4 sont, de préférence, de longueur réglable. Sur l'axe X-X′, devant la plateau 1, est montée une poulie 5 sur la gorge de laquelle passe une ficelle 6 entraînant la rotation d'un potentiomètre 7. Les rotations du potentiomètre 7 recopient ainsi algébriquement celles du plateau 1 autour de l'axe X-X′. les côtés du plateau 1 parallèles à l'axe X-X′ et les côtés du socle 3 parallèles à l'axe X-X′ peuvent être reliés par des ressorts qui augmentent la stabilité du plateau 1. Ces ressorts peuvent être constitués par des tendeurs élastiques réglables.

Dans l'exemple de la Fig. 1, près de l'ensemble 1-2-3, est placée une bascule électronique 8. Les positions relatives de l'ensemble 1-2-3 et de la bascule 8 sont telles qu'une personne peut avoir un pied, ici le pied droit, sur l'ensemble 1-2-3 et l'autre pied, ici le pied gauche sur la bascule 8, avec une attitude debout tout à fait normale. Il peut faire porter plus de poids sur un pied ou l'autre.

Les fils 9 du potentiomètre 7 sont reliés à l'entrée d'un convertisseur analogique-numérique 10. La sortie du convertisseur 10 et la sortie 11 de la bascule 8 sont respectivement reliées aux entrées 12 et 13 d'une mémoire 14 faisant partie d'un microordinateur 15. Celui-ci comprend une source de courant continu, non montrée, permettant d'alimenter le potentiomètre 7.

Le microordinateur 15, outre son unité logique de commande centrale non montrée, comporte une base de temps 16 et un dispositif d'affichage 17 sous la forme d'un écran de tube cathodique. La base de temps 16 synchronise les fonctionnements de la mémoire 14 et du dispositif d'affichage 17.

Sous la commande de l'unité logique du microordinateur 15, les données enregistrées dans la mémoire 14 et provenant simultanément du potentiomètre 7 et de la bascule 8 peuvent être affichées sous la forme de deux courbes.

L'appareil de l'invention est, de préférence, complété par un dispositif de mesure de potentiels 18 pourvu de sondes 19, auxquelles il est relié par des fils. Un tel dispositif est classique. Les sondes sont appliquées sur des muscles, par exemple les péroniers latéraux. Suivant l'état de travail du muscle, l'électrode correspondante recueille un potentiel différent qui est détecté et mesuré dans le dispositif 19 et numérisé. La sortie du dispositif 18 est reliée à l'entrée 20 de la mémoire 14.

En pratique, le dispositif 18 sert à observer, par exemple, l'activité des péroniers au cours du mouvement d'une cheville.

Quand on utilise le matériel montré à la Fig. 1, on suppose que, par exemple, le pied gauche est le pied valide et le pied droit le pied pathologique. Dans ce cas, il est évident que le patient tend à mettre tout son poids, ou au moins plus de poids, sur le pied posé sur la bascule 8. C'est pratiquement la position initiale. Le rééducateur demande au patient de porter plus de poids sur le plateau 1 dont la position est instable autour de l'axe X-X′. Pendant un premier temps, le patient parvient à compenser cette instabilité en ramenant le plateau 1 à sa position horizontale dès qu'il s'en écarte. Puis, plus tard, le poids appliqué sur le plateau 1 augmentant, l'instabilité se traduit par une brusque inclinaison du plateau, jusqu'à ce qu'il rencontre le sommet des butées 4, d'un côté ou de l'autre. Le patient peut alors ramener la majorité du poids sur la bascule 8. Pendant cette phase, les mouvements angulaires du plateau ont été enregistrés à l'aide du potentiomètre 7 et, simultanément, le poids restant appliqué sur la bascule est également enregistré. Chaque phase dure environ ?? minutes. On sait, en observant la courbe du poids, quand le patient a commencé à appuyer sur le plateau 1. On sait quand il a cessé de contrôler le plateau et quel était alors le poids qu'il y appliquait. L'examen des courbes permet au rééducateur d'en tirer des conclusions.

Les données délivrées par le dispositif 18 permettent également de savoir quand les muscles ont commencé à agir, après le début du déséquilibre, et quand ils ont cessé d'agir, après le retour à l'équilibre.

On peut retourner le patient de manière qu'il ait le bon pied sur le plateau et le mauvais pied sur la bascule. Les mesures réalisées, comme mentionné ci-dessus, permettent d'évaluer la qualité du bon pied, sachant que cette qualité définira le but à atteindre pour le pied rééduqué.

## Revendications

1. Appareil d'étude et de rééducation de la cheville caractérisé en ce qu'il comporte un plateau (1) qui peut pivoter sur un axe horizontal (X-X′) monté sur des potelets (2) dont les pieds sont fixés sur un socle (3), d'une part, et une bascule (8), d'autre part, une personne pouvant avoir un pied sur le plateau (1) et l'autre pied sur la bascule (8), des butées (4) limitant l'inclinaison du plateau (1) de part et d'autre de l'axe, des moyens de mesure (7) de l'inclinaison du plateau, une première mémoire (14) des valeurs délivrées par lesdits moyens de mesure (7), la sortie de la bascule (8) étant reliée à une deuxième mémoire (14) de données numériques, des moyens d'affichage (17) étant reliés simultanément aux sorties de la première et de la deuxième mémoire de données.

2. Appareil suivant la revendication 1, caractérisé en ce que les moyens de mesure sont un potentiomètre électrique (7) asservi au mouvement du plateau (1) autour de son axe de pivotement (X-X′) et associé à un appareil de mesure de courant ou de tension électrique dont la sortie est reliée à un convertisseur analogique-numérique (10) dont la sortie est reliée à l'entrée (12) de la première mémoire (14).

3. Appareil suivant la revendication 1 ou 2, caractérisé en ce qu'il comprend encore un dispositif de mesure numérique (18) de potentiels électriques recueillis par des sondes (19) appliqués sur des muscles, particulièrement les péroniers latéraux, la sortie numérique du dispositif (18) étant reliée à une troisième mémoire (14) de données numériques, les moyens d'affichage (17) pouvant être reliés simultanément sélectivement aux sorties des première, deuxième et troisième mémoires.

4. Appareil suivant l'une des revendications 1 à 3, caractérisé en ce que les moyens d'affichage font partie d'un microordinateur (15) qui comprend aussi une mémoire (14) comportant trois zones de mémoire représentant les première, deuxième et troisième mémoires respectivement, une base de temps et une unité de commande centrale, les moyens d'affichage étant formés par l'écran (17) du microordinateur (15).

## Patentansprüche

1. Vorrichtung zum Training der Fußknöchel, **dadurch gekenn****zeichnet,** daß sie folgendes enthält:
einerseits eine Platte (1), die um eine horizontale Achse (X - X′) schwenkbar ist, die auf Pfosten (2) gelagert ist, deren Füße auf einem Sockel (3) befestigt sind, und andererseits ein Schaukelbrett (8), wobei eine Person einen Fuß auf die Platte (1) und den anderen Fuß auf das Schaukelbrett (8) setzen kann, außerdem Stützen (4), die die Neigung der Platte (1) in beiden Richtungen zur Achse begrenzen, ferner Meßmittel (7) für die Neigung der Platte, ferner einen ersten Speicher (14) für die von den Meßmitteln (7) gelieferten Werte, wobei der Ausgang des Schaukelbrettes (8) mit einem zweiten digitalen Datenspeicher (14) verbunden ist, und ferner Anzeigemittel (17), die gleichzeitig mit den Ausgängen des ersten und des zweiten Datenspeichers verbunden sind.

2. Vorrichtung nach Anspruch 1, **dadurch gekennzeichnet,** daß die Meßmittel durch ein elektrisches Potentiometer (7) gebildet sind, das durch die Bewegung der Platte (1) um seine Schwenkachse (X - X′) gesteuert und an ein elektrisches Strom- oder Spannungsmeßgerät angeschlossen ist, dessen Ausgang an einen Analog/Digital-Wandler (10) angeschlossen ist, dessen Ausgang an den Eingang (12) des ersten Speichers (14) angeschlossen ist.

3. Vorrichtung nach Anspruch 1 oder 2, **dadurch gekennzeichnet,** daß sie außerdem ein digitales Meßgerät (18) für elektrische Spannungen enthält, die von Sonden (19) aufgenommen werden, die auf Muskeln, insbesondere Wadenmuskeln, angewendet werden, und daß der digitale Ausgang des Gerätes (18) mit einem dritten digitalen Datenspeicher (14) verbunden ist und die Anzeigemittel (17) gleichzeitig einzeln mit den Ausgängen des ersten, zweiten und dritten Speichers verbunden werden können.

4. Vorrichtung nach einem der Ansprüche 1 - 3, **dadurch** **gekennzeichnet,** daß die Anzeigemittel ein Teil eines Mikroprozessors (15) bilden, der außerdem einen Speicher (14) mit drei Speicherbereichen, die den ersten, zweiten und dritten Speicher darstellen, einen Zeitgeber und eine zentrale Steuereinheit enthält, wobei die Anzeigemittel durch den Bildschirm (17) des Mikroprozessors (15) gebildet sind.

## Claims

1. Ankle study and re-education apparatus, characterised in that it comprises a plate (1) which can pivot on a horizontal axle (X-X′) mounted on struts (2) the feet of which are fixed to a base (3) on the one hand and a rocker (8) on the other hand, a person being able to have one foot on the plate (1) and the other foot on the rocker (8), stops (4) limiting the slope of the plate (1) on both sides of the axle, means for measuring (7) the slope of the plate, a first memory (14) of the values supplied by the said measuring means (7), the output of the rocker (8) being connected to a second memory (14) of numerical data, display means (17) being connected simultaneously to the outputs of the first and second data memory.

2. Apparatus according to claim 1, characterised in that the measuring means are an electric potentiometer (7) served by the movement of the plate (1) around its pivot axle (X-X′ and associated with an electric current or voltage measuring apparatus whose output is connected to an analogical-numerical converter (10) whose output is connected to the input (12) of the first memory (14).

3. Apparatus according to claim 1 or 2, chracterised in that it comprises also a numerical measuring device (18) of electric potentials collected by probes (19) applied to the muscles particularly the lateral peroneal the numerical output of the device (18) being connected to a third memory (14) of numerical data, the display means (17) being capable of being connected simultaneously selectively to the outputs of the first, second and third memories.

4. Apparatus according to one of the claims 1 to 3, characterised in that the display means form part of a microcomputer (15) which also comprises a memory (14) having three memory zones representing the first, second and third memories respectively, a time base and a central control unit, the display means being formed by the screen (17) of the microcomputer (15).
